# EUROPEAN PATENT SPECIFICATION

(11) **EP 0 805 696 B1**
(45) Date of publication and mention of the grant of the patent: **15.03.2000**
(21) Application number: 96900294.8
(22) Date of filing: 22.01.1996
(51) Int. Cl.: A61M 15/00

(54) **AN INHALER**
INHALATOR
INHALATEUR

(30) Priority: 23.01.1995 DK 8295
(43) Date of publication of application: 12.11.1997
(73) Proprietor: Direct-Haler A/S, 5250 Odense SV (DK)
(72) Inventor: KELDMANN, Erik, DK-5250 Odense SV (DK); REIPUR, John, DK-2930 Klampenborg (DK)
(74) Representative: Siiger, Joergen
(86) International application number: DK9600034
(87) International publication number: WO9622802

(56) References cited:
- EP-A- 0 404 454
- WO-A-89/01348
- WO-A-93/17728
- WO-A-94/05358
- US-A- 2 503 732
- US-A- 4 148 308
- US-A- 4 265 236

## Description

The present invention relates to an inhaler of the type comprising a tubular body defining an air flow passage therein.

Numerous inhalers of this type are known. As examples, US patent Nos. 4,524,769 and 4,907,583 and the international application WO 90/07351 disclose inhalers each comprising dosing means for supplying a dose of a particulate active substance to the air flow passage when the inhaler is to be used. The outlet end of the discharge passage is defined by a mouthpiece or nozzle which during use of the inhaler is positioned between the lips of the user. These known inhalers are of a relatively complicated structure, and they are not simple in use because the dosing means has to be operated each time the inhaler is to be used. Furthermore, these known devices are relatively heavy and bulky to carry in a pocket or in a ladies handbag.

When a particulate or powdered substance is inhaled through a mouthpiece or nozzle positioned between the users lips a substantial part of the active substance suspended in the inhaled air flow will not reach the lungs of the user, but may be swallowed or may impinge the mucous membrane of the oral cavity of the user. This does not only mean that a substantial amount of the active substance is lost, but also that the active substance coming into contact with the mucous membrane of the oral cavity or arriving at the stomach may have undesired side effects.

As disclosed in German Offenlegungsschrift No. 2815039 this problem may be solved by using a mouthpiece which in use extends into the oral cavity and along the tongue of the user. When a fluid flow including an active substance is directed into the oral cavity of the patient, for example from an inhaler or an atomizing device connected to the outer end of the mouthpiece, it is possible to direct such fluid flow towards a desired part of the oral cavity, such as the throat or trachea of the patient.

US-A-2 503 732 discloses an inhaler comprising a straight tube having a valved stopper inserted in one end and a mouth- or nosepiece fitted to the other end thereof. WO-A-9 317 728 relates to a disposable inhaler made from a part being a moulded sheet of plastic material and an opposite part made from a plane sheet material. These parts form a tubular housing with a compartment for powder to be inhaled and a constriction causing turbulence in an air stream through the housing. EP-A-0 404 454 also discloses a disposable medicament inhalation device having a flat chamber, which is defined between a flat lower part and a moulded upper part heat sealed together. An inlet opening and a plurality of outlet openings are formed in the upper part. US-A-4 265 236 relates to an inhaler comprising a length of flexible tubing which contains a flow control device. The length of tubing may be formed into a closed loop whereby the ends of the tubing are closed.

The present invention provides an inhaler which may be produced in a very simple manner and at low cost and which is nevertheless very efficient.

The inhaler according to the present invention comprises a one piece tubular body, which has a mouthpiece section and is intended to be used only once, and which defines an air flow passage therein, the tubular body having substantially rectilinear sections, and a single dose only of an active inhalable, particulate substance being arranged within the air flow passage, said dose being sealed or closed in relation to the ambient atmosphere by closure means which are to be removed or opened by a user prior to use.

An inhaler of this type is known from WO-A-8 901 348. This publication discloses a disposable powder inhaler formed as a straight tube sealed at both ends and containing a dose of the powder to be inhaled. When the inhaler is to be used by a patient the sealed ends of the tube are opened, and one end of the tube may then be inserted into the mouth or nose of the patient. Thereafter, the powdered substance may be inhaled.

The inhaler according to the invention is characterised in that the tubular body comprises a curved and/or bendable section intermediate of the rectilinear sections, and in that the cross-sectional area of the flow passage defined in the tubular body does not exceed 75 mm2.

The said closure means may be of any suitable type, such as caps, films, or foils which are removably arranged at the opposite ends of the tubular body. Alternatively, the closure means may comprise removable, puncturable or rupturable membranes extending across the air flow passage and being axially spaced therein so that the dose of active substance is arranged therebetween. The closure means could, of course, be of any other type, which is able to seal the dose of active substance in relation to the ambient atmosphere until the inhaler is to be used, and which may be removed or opened by a patient or user prior to use.

The patient patient may now inhale the active substance by inserting the mouthpiece end between the lips and by forcefully inhaling air through the air flow passage defined in the inhaler. Because the cross-sectional area of the inner section of the air flow passage is relatively small, the air velocity in this section will be relatively high. Such high air velocity promotes atomization of the dose of particulate active substance and suspension of the finely divided particles in the air flow.

In order to obtain a high air flow rate the cross-sectional area of the said flow passage section preferably does not exceed 70 mm² and is more preferably less than 50 mm². In the presently preferred embodiments the cross-sectional area of the flow passage section is 7-35 mm² and preferably about 20 mm².

The flow passage or the flow passage section may have any cross-sectional shape, such as square, rectangular, polygonal, elliptical, or circular. Furthermore, the cross-sectional area of the flow passage or the flow passage section may vary along the length thereof. Thus, the cross-sectional area of the flow passage or flow passage section may be smaller at the mouth piece end than at the opposite end of the flow passage. In the preferred embodiment, however, the flow passage or flow passage section has a substantially circular cross-section, the inner diameter of the flow passage section, which may be the total length of the air flow passage, being substantially the same along the length of the section. Thus, in a very simple embodiment the inhaler is formed similar to a drinking straw.

The tubular body of the inhaler may have a predetermined, permanent shape which cannot be changed by the user. In a preferred embodiment the tubular body comprises at least one bendable section so that the shape of the tubular body may be adapted to the form of the oral cavity of the individual user. The walls of the tubular body forming the bendable section may be made from a deformable plastic material. Alternatively, the flexibility of the bendable section may be obtained by providing the bendable section of the tubular body with peripherally extending corrugations. The bottoms of these corrugations may be provided with codes, such as colours, numbers, letters, or other kinds of indications, for assisting in obtaining a bend suitable for the individual user. When a patient or user has determined a shape of the tubular body which has been adapted to his individual oral cavity, the user may read and note the visible code combination. When the user knows his individual code combination he may quickly adjust the bendable section of the tubular inhaler body next time he is using an inhaler of the same type.

In order to improve the dispersion of the particulate active substance in the air flowing through the air flow passage the inhaler may further comprise means for imparting a rotational movement to the air about the longitudinal axis of the flow passage. Such means may, for example, be helical grooves or ribs formed on the wall surface parts of the tubular body defining the flow passage, or rotation imparting members arranged centrally within the air flow passage.

The tubular body of the inhaler may comprise a mouth piece section of any suitable length extending during use from the teeth of the user to any desired position within the oral cavity of the user. Thus, by suitably adapting the length and the shape of the mouthpiece section the inner end of the air flow passage may be positioned adjacent to and may be directed towards any part of the oral cavity which is to be treated by means of the active particulate substance. In cases where the active substance is to be inhaled to the lungs or bronchial tubes of the user or patient, for example when the active substance is a medicament for alleviating asthmatic deceases, the mouthpiece section of the tubular body preferably has a length so as to extend during use from the teeth of the user to a position adjacent to the root of the user's tongue. The mouthpiece section may then advantageously be shaped so that the inner end of the air flow passage is directed towards the throat or trachea inlet of the patient or user. Thereby it may be secured that substantially all of the active substance of the dose positioned in or supplied to the air flow passage will reach its target area.

In order to secure that the inhaler is correctly positioned within the oral cavity of the user or patient the inhaler may further comprise a bite piece formed on the outer surface of the tubular body for engaging with the upper jaw teeth of the user. Such bite piece may form an integral part of the tubular body. It is preferred, however, that the shape of the bite piece is adapted to the teeth of the individual user. In such case the relatively expensive and individually formed bite piece is removably mounted on the tubular body of the inhaler so that the bite piece may be kept and reused when the tubular body is discarded. Such removably mounted bite piece may advantageously be used in connection with tubular bodies adapted to be used only a few times or only once. The bite piece may be combined with a set of false teeth. Thus, such a set of false teeth may comprise means for positioning the tubular body in relation to the oral cavity of the user, or the bite piece may be individually shaped when the false teeth are made to the individual user.

The tubular body of the inhaler may have a fixed length. Alternatively, the tubular body may be movable from a retracted storage condition to an extended condition of use. This may, for example, be obtained by providing the tubular body with peripheral corrugations along a major part of its length so as to allow longitudinal stretching of the tubular body. As another possibility, the tubular body may comprise telescopically cooperating tubular parts allowing such parts to be moved between retracted and extended positions. The closure means sealing the dose of active substance in relation to the ambient atmosphere or the supply means for supplying such dose into the air flow passage of the inhaler may be opened or actuated automatically when the tubular body is moved from its retracted storage condition to its extended condition of use.

In order to completely eliminate the risk that larger particles of the active substance or of foreign matter, such as ruptured or removed closing means, are inhaled by a patient the inhaler may further comprise retaining means arranged within the flow passage for retaining particles of a size exceeding a predetermined size. Such retaining means may, for example, comprise a sieve or screen extending across the flow passage and being arranged downstream of the dose of active substance to be inhaled. Alternatively, when the closure means comprise removable closure caps, such caps may be integrally connected to the tubular body by flexible strings or bands. Alternatively or additionally, the free end portions of the tubular body may be bent towards each other so as to position the free ends closely adjacent, and the free ends may then be closed by a common, removable closure member, such as a pair of interconnected or integrally formed closure caps. Furthermore, at least one of the cap members is preferably made from a transparent material, or the tubular body is at least partly made from a transparent material, so that the patient or user may see that the tubular body contains a dose of the active substance.

In most cases the patient or user is able to inhale atmospheric air through the air flow passage so as to obtain an air flow being sufficiently vigorous to suspend the active substance therein in an atomized condition. However, smaller children and adult persons having a substantially reduced lung capacity may not be able to generate a sufficient air flow rate through the air flow passage. Therefore, the inhaler may further comprise forced flow generating means for providing a forced air flow through the flow passage. Such flow generating means may be of any kind which is able to generate pressurized air. As an example, the forced flow generating means may comprise a compressible bulb to be mounted on the outer end or air inlet end of the tubular body. The flow generating means may then be actuated at the same time as the patient is inhaling.
It should be understood that the inhaler according to the present invention should not necessarily be inserted into the oral cavity of the user, and the term "mouthpiece" is not intended to indicate that it should necessarily be inserted into the user's mouth. Thus, one end or the mouthpiece end of the tubular body may be adapted to be inserted into a nostril of a user or patient. In such case, the inhaler preferably comprises a pair of tubular bodies and a connecting part for interconnecting the same, said one end or mouthpiece end of said pair of tubular bodies being arranged in spaced relationship, so that said ends may be inserted into the nostrils of a user or patient.

Preferred embodiments of the invention will now be further described with reference to the drawings, wherein
Fig. 1 illustrates a first embodiment of the inhaler according to the invention,
Fig. 2 illustrates a second embodiment of the inhaler according to the invention,
Fig. 3 illustrates the use of the inhaler shown in Fig. 1,
Fig. 4 illustrates a third embodiment of the inhaler according to the invention,
Fig. 5 illustrates a fourth embodiment of the inhaler according to the invention,
Fig. 6 illustrates how the inhaler shown in Fig. 5 may be used,
Fig. 7 illustrates how a bent portion of the inhaler shown in

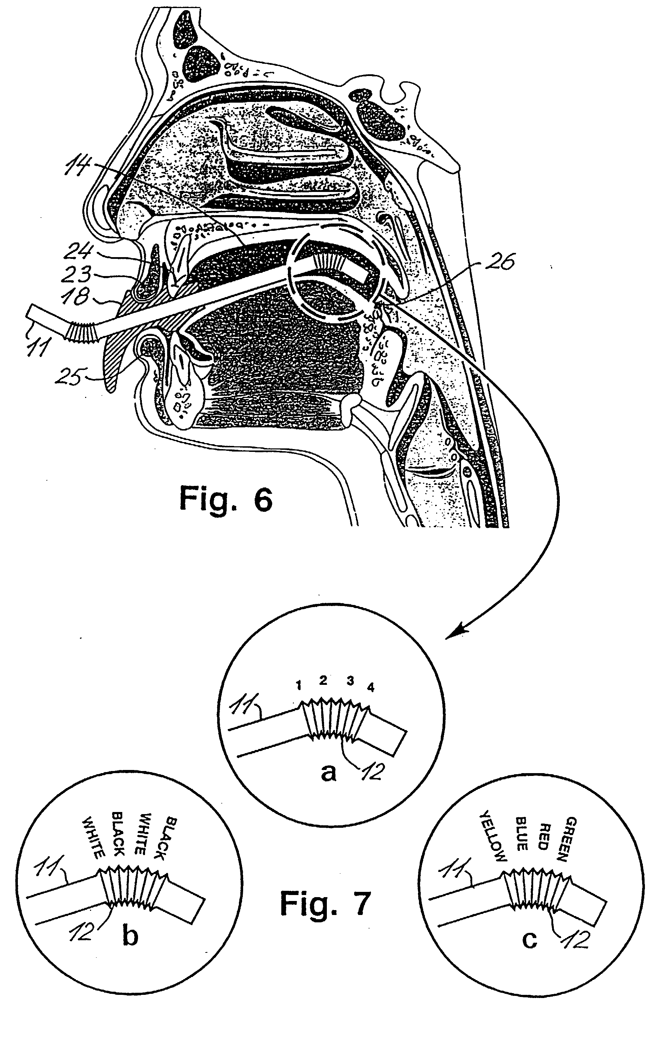

Fig. 6 may be provided with bending codes,

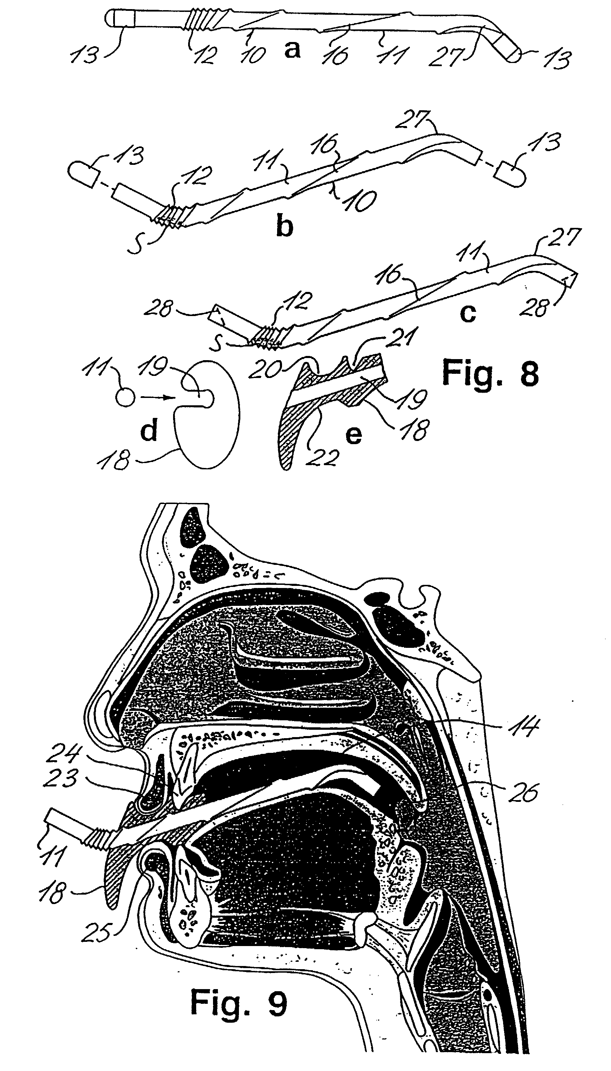

Fig. 8 illustrates a fifth embodiment of the inhaler according to the invention,
Fig. 9 illustrates how the inhaler shown in Fig. 8 may be used,

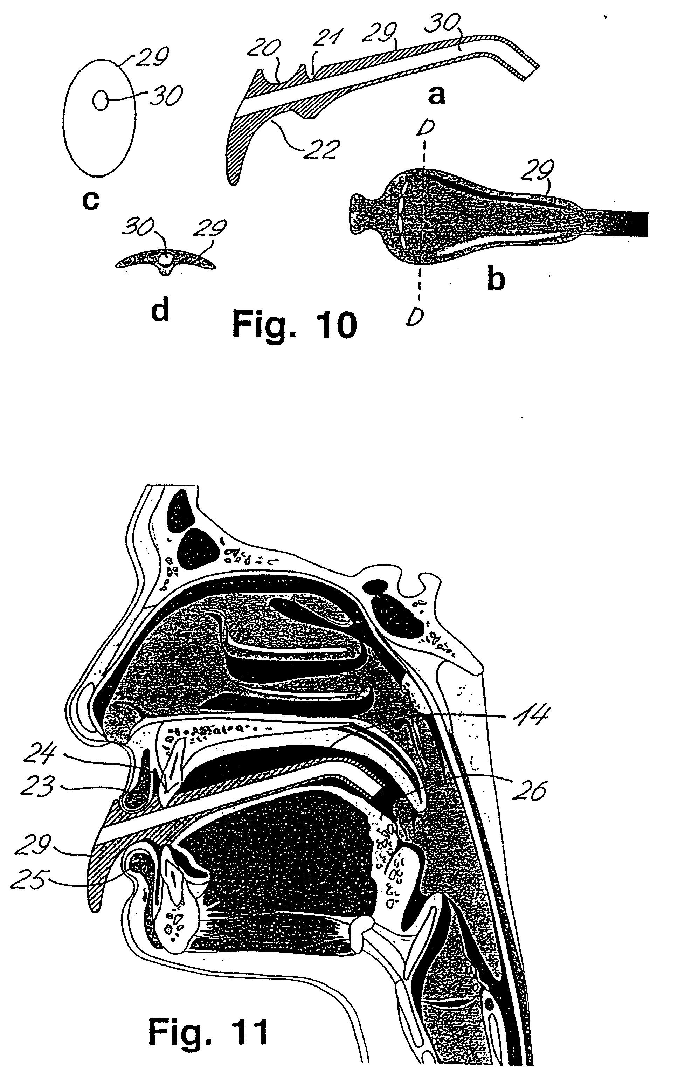

Fig. 10 illustrates a mouthpiece for a sixth embodiment of the inhaler according to the invention,
Fig. 11 illustrates how the mouthpiece shown in Fig. 10 may be positioned in the oral cavity of the user,

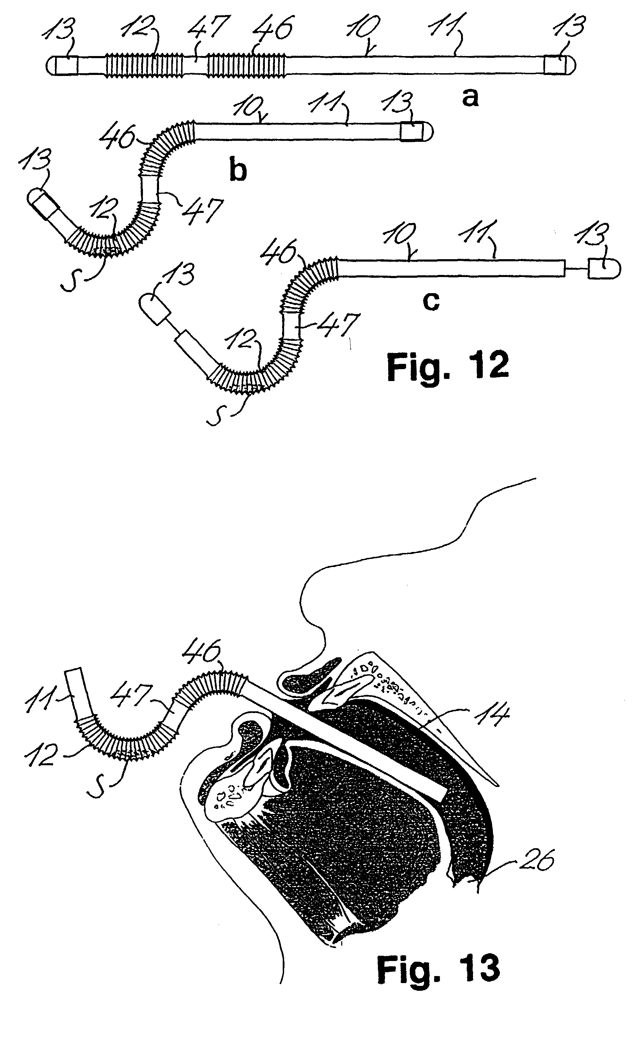

Fig. 12 illustrates a seventh embodiment of the inhaler according to the invention,
Fig. 13 illustrates how the inhaler shown in Fig. 12 may be used,

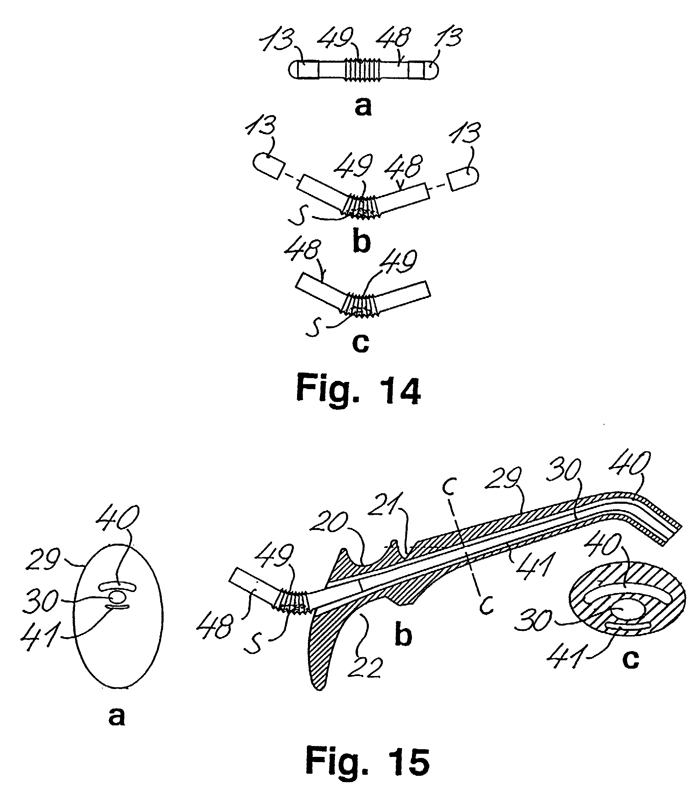

Fig. 14 shows a capsule containing a single dose of an active substance,
Fig. 15 illustrates how the capsule shown in Fig. 14 may be used in connection with a mouthpiece as shown in Figs. 14-16

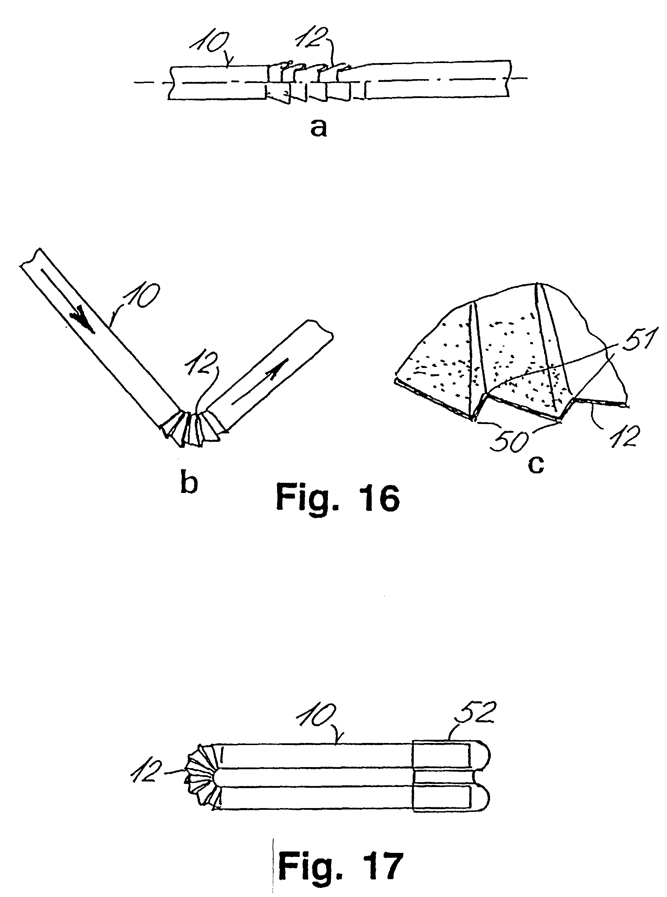

Fig. 16 shows an eighth embodiment of the inhaler, and
Fig. 17 shows a nineth embodiment, in which the opposite ends of the tubular inhaler is closed by a common closure cap.

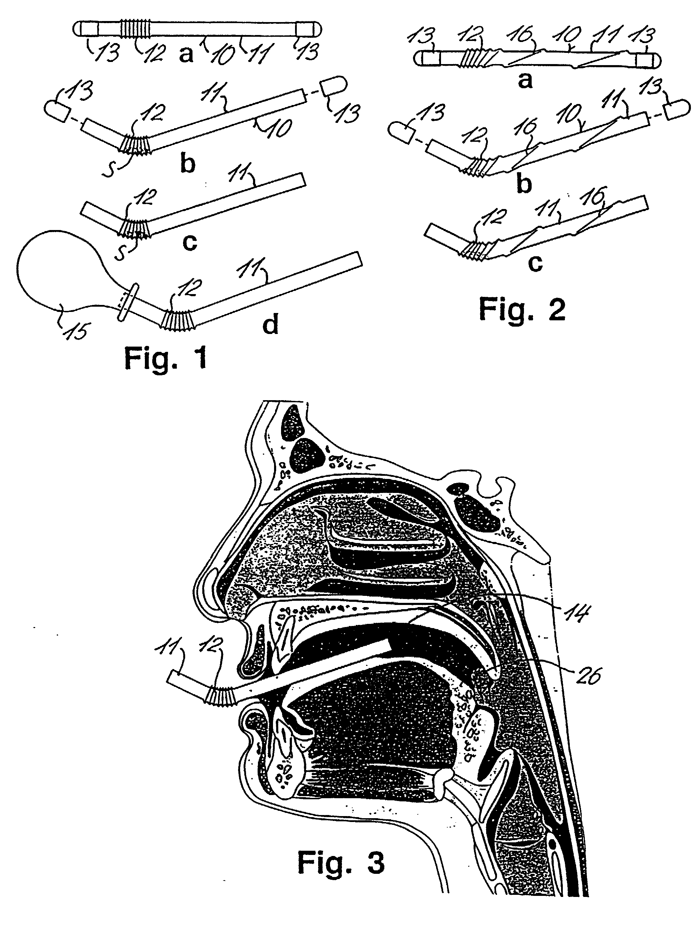

Fig. 1 shows an inhaler 10. When delivered from the manufacturer, the inhaler may comprise a straight, thin-walled tubular body 11 having a bendable section 12 and removable caps 13 closing the opposite open ends of the tubular body 11. The inner bore of the tubular body 11 which defines an air flow passage of the inhaler contains a single dose of a particulate or powdered active substance, such as steroids, β₂-agonists, anticholinergica, or other medical products. The tubular body 11 may have a circular cross-sectional shape and have a substantially uniform inner diameter and wall thickness along the length of the tubular body and may be similar to a drinking straw. The section 12 may have peripheral corrugations so as to be bendable. The tubular body 11 may, for example, be made from a suitable plastic material by extrusion, and the inner diameter of the tubular body is preferably within the range of 4-8 mm, for example 5-6 mm. The material of the tubular body 11 may have been treated so as to reduce or eliminate the possibility of static electricity.

The inhaler 10 shown in Fig. 1a which contains only a single dose of the active substances is intended to be used only once whereafter the inhaler is discarded. A suitable small number of disposable inhalers of this type may be packed, for example similar to cigarettes, and they may be carried by the user in a pocket or a ladies handbag without occupying much space.

When an inhaler 10 of the type shown in Fig. 1a is to be used the user or patient may shake the inhaler so as to disintegrate the particulate active substances contained therein. The bendable section 12 may now be bent whereby the active substance S is positioned within the corrugation troughs of the bendable section 12 as indicated in Figs. 1a and 1b. Thereafter the caps 13 may be removed as shown in Fig. 1b. The inhaler 10 is then ready for use, and a longer straight end portion of the tubular body may be inserted into the oral cavity 14 of a user or patient as shown in Fig. 3. Because the tubular body 11 has been bent the active substance S contained in the corrugation troughs of the section 12 may be prevented from falling out from the tubular body.

As shown in Fig. 3 the inner end of the inhaler is positioned adjacent to the root of the patient's tongue. When the patient inhales air through the air flow passage defined within the tubular body 11 the particulate active substance S is withdrawn from the corrugation troughs of the section 12 and is suspended in the air flow which is inhaled into the patient's lungs. In case the patient is a small child or for some other reason is not able to inhale sufficiently vigorously a compressible bulb 15 or other means for generating a forced flow through the tubular body 11 may be mounted on the outer end thereof as shown in Fig. 1d. A flow of air with active substance suspended therein may then be blown into the oral cavity 14 at the same time as when the patient inhales. The embodiment shown in Fig. 2 is slightly modified in relation to the embodiment shown in Fig. 1. Thus, in Fig. 2 the tubular body 11 is provided with a helically extending corrugation 16 which may impart a rotational movement to the air flow being inhaled through the air flow passage defined in the tubular body 11. The tubular body 11 and/or at least one of the closure caps 13 is preferably made from a transparent material so that the user may visually make sure that the inhaler contains a dose of an active substance.

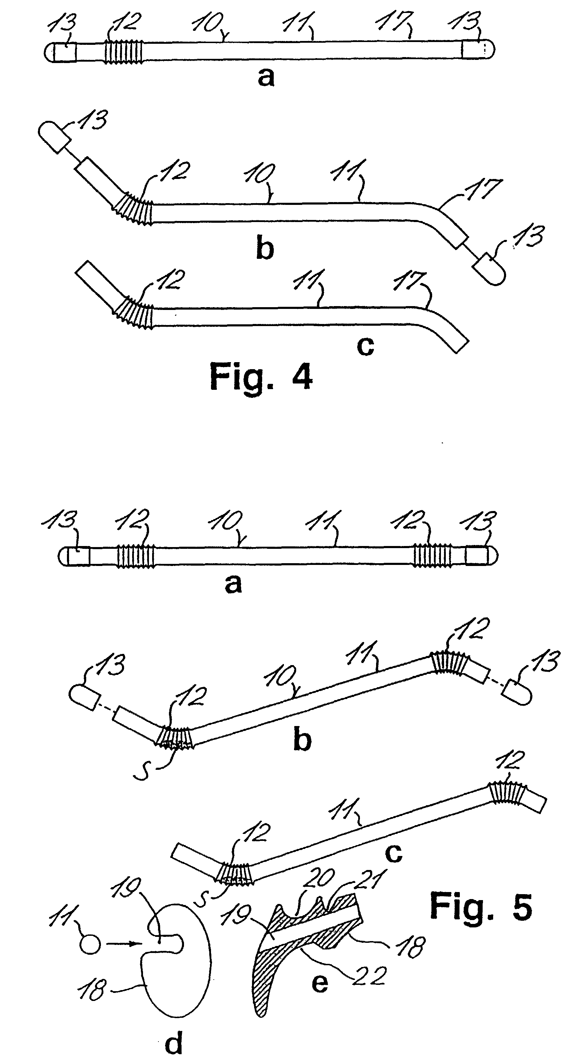

Fig. 4 shows an embodiment 10 having not only a bendable section 12 provided with peripherally extending corrugations, but also a bendable section 17 without such corrugations at an opposite end portion of the tubular body 11. In fact, the material and the wall thickness of the tubular body 11 may be chosen so that any part of the tubular body may be bent into a desired shape. The inhaler shown in Fig. 5 corresponds to that shown in Fig. 4. The only difference is that the embodiment shown in Fig. 5 comprises a bendable section 12 at opposite end portions of the tubular body, which sections 12 both comprise peripherally extending corrugations.

For some patients who are not sensing so well it may be difficult to immediately position the inhalers shown in Figs. 1, 2, 4, and 5 correctly in the oral cavity 14. Therefore, the tubular body 11 may advantageously be inserted in a bite piece or teeth block 18 of the type shown in Fig. 5. Fig 5d is an end view of the bite piece 18, while Fig. 5e is a longitudinally sectional view of the bite piece. The bite piece 18 comprises a longitudinally extending channel or slot 19 which is dimensioned so that the tubular body 11 may be snugly received therein as indicated by an arrow in Fig. 5d. Troughs or grooves 20 and 21 are defined in the upper outer surface of the bite piece 18, and a trough 22 is defined in the lower outer surface of the bite piece. When the bite piece or teeth block 18 has been mounted on the tubular body 11 which has been bent into the desired shape and the closure caps 13 have been removed as explained above, the inhaler assembly comprising the tubular body 11 and the bite piece 18 may be inserted into the user's mouth. As shown in Fig. 6 the bite piece or teeth block 18 may then be positioned so that the upper lip 23 and the upper teeth 24 of the patient are positioned in the troughs 20 and 21, respectively, while the lower lip 25 of the user is positioned in the trough 22 of the bite piece or teeth block 18 whereby the tubular body 11 may be positioned very accurately within the user's oral cavity 14.

As shown in Fig. 6 the inner end portion of the tubular body 11 may be shaped so that the inner open end of the tubular body is positioned adjacent to and directed towards the throat 26 of the user or patient. Thus, almost all of the active substance contained within the tubular body may be transferred to the patient's lungs when the patient vigorously inhales air through the air flow passage defined within the tubular body 11. It should be understood that the inner end portion of the tubular body 11 could be directed towards any desired surface part of the oral cavity to be treated by the active substance contained in the inhaler.

In order to ensure that the open inner end of the tubular body 11 is directed towards the throat 26 of the patient or user as shown in Fig. 6, or towards any other surface part of the oral cavity 14 to be treated it is important that the shape of the inner end of the tubular body 11 is adapted to each individual user. When the inner end of the tubular body has a bendable section 12 provided with peripherally extending corrugations, the bend or curvature which is adapted to the oral cavity of a specific patient or user may be expressed as a code which may be remembered. Fig. 7 illustrates examples of such coding. As indicated in Fig. 7a a number may be assigned to each or every second of the peripheral troughs formed between the adjacent peripheral corrugations of the bendable section 12. The number code to be remembered by the user may then indicate which of the upwardly facing trough parts should be fully opened and which should not.

Alternatively, adjacent troughs may be differently coloured or different colours may otherwise be assigned to the various troughs as indicated in Figs. 7b and 7c. The bend or curvature suitable for each individual user or patient may then be expressed as a colour code in a similar manner as explained above in connection with the number coding.

The inhaler 10 illustrated in Fig. 8 is of the same type as that described above with reference to Fig. 2. However, the inner end portion of the tubular body 11 shown in Fig. 8 has a permanent bend 27 which is made when the inhaler is being manufactured.

Like in the inhaler shown in Fig. 2 the tubular body 11 has a helical corrugation 16 extending along the length of the tubular body. As explained above in connection with Fig. 2, such helical corrugation may tend to impart a rotational movement to air inhaled through the tubular body 11. The tubular body 11 in any of the embodiments shown in Figs. 1, 2, 4, 5 and 8 may, for example, be made by injection moulding, blow moulding, or extrusion. In the latter case, the corrugations may be formed in the walls of the tubular body 11 during a subsequent manufacturing stage. The tubular bodies 11 shown in Figs. 2 and 8 may, alternatively, be made by helically winding a strip of sheet material, such as paper, paperboard or another fibrous sheet material, with mutually overlapping adjacent edge portions. Such edge portions may then be interconnected or sealed, whereby the helically extending corrugation 16 may be formed. In order to facilitate mounting of the snugly fitting caps 13 on the opposite ends of the tubular body 11 when a dose of active material has been arranged therein, the tubular bodies 11 illustrated in Figs. 1, 2, 4, 5, and 8 may be cut on the bias as indicated with dotted lines at 28 in Fig. 8c.

The tubular body 11 shown in Fig. 8 may be inserted into the channel or slot 19 of a bite piece or teeth block 18 as explained above in connection with Fig. 5. Thereafter, when the bendable section 12 has been given the desired shape and the caps 13 have been removed, the inhaler may be positioned in the mouth of a patient as illustrated in Fig. 9 and as explained above in connection with Fig. 6.

Fig. 10 illustrates an embodiment of a tubular body 29 for an inhaler according to the invention. Figs. 10a, 10b, 10c, and 10d illustrate a longitudinally sectional view, a top plan view, an end view, and a cross-sectional view along the line D-D, respectively. The tubular body 29 defines a longitudinally extending air flow passage 30 having a substantially uniform cross-sectional area along the length thereof. The outer end (the left hand end in Fig. 10) of the tubular body 29 has a shape corresponding to the shape of the bite piece or teeth block 18 shown in Figs. 5 and 8. This means that in the embodiment shown in Fig. 10 the bite block is formed integrally with the tubular body 29. Thus, the tubular body 29 has troughs or grooves 20 and 21 formed in the upper outer surface of the outer end portion of the tubular body 29. These troughs 20 and 21 are intended to receive the upper lip 23 and the upper teeth 24, respectively, of a user or patient. Furthermore, a trough 22 is formed in the lower outer surface of the tubular body 29 for receiving the lower lip 25 of the user as illustrated in Fig. 11.

The tubular body 29 illustrated in Fig. 10, which is preferably made from plastic by injection moulding, may be adapted to be used only once. In such case, a single dose of an active powdered or particulate material may be arranged within the air flow passage 30, and the open ends of the air flow passage may be sealed or closed by removable sealing or closing means, such as a film or foil which may be torn off. Alternatively, the tubular body 29 illustrated in Fig. 10 may be adapted to cooperate with a dose feeding device for feeding a dose of powdered or particulate active material into the air flow passage 30 when the inhaler is to be used. In that case the tubular body 29 is preferably adapted to be used several times, and each sample may then have a shape which has been adapted to the individual user.

In Fig. 12 an embodiment corresponding to that of Fig. 1 has been shown. However, in addition to the bendable section 12 the tubular body 11 has a second similar bendable section 46 which is spaced from the bendable section 12 by a non-corrugated, rectilinear tubular section 47. The tubular body 11 contains a single dose of an active substance S and when the inhaler 10 is to be used the sections 12 and 46 may be bent as shown in Figs. 12b and 12c so that the sections 12, 47 and 46 are substantially S-shaped. The active substance S is mainly received in the inner corrugation troughs of the bendable tubular section 12. Now, the removable closure caps 13 may be removed from the opposite ends of the tubular body 11 as illustrated in Fig. 12c, and the tubular body may be inserted into the oral cavity 14 of a user as illustrated in Fig. 13. Even when the user reclines his head as shown in Fig. 13 the active substance S may remain in the inner corrugation troughs of the bendable section 12. When, however, the user or patient inhales air through the air flow passage of the tubular body 11, the velocity of flowing air causes a static pressure drop so that the active substance S is sucked from the corrugation troughs and entrained with and efficiently dispersed in the air flow.

If an inhaler as that shown in Figs. 1, 2, and 4 is shortened it may be used as a disposable container or capsule for a single dose of an active substance S. Such a tubular container or capsule is shown in Fig. 14 and may be closed at its opposite ends by removable closure caps 13 or by any other removable or breakable closure means. The tubular capsule may have a central bendable section 49 having peripheral corrugations as those previously described. The tubular container or capsule 48 may be used together with the tubular body 29 shown in Fig. 10. Fig. 15 illustrates how the container or capsule 48 may be used in connection with a tubular body 29 having upper and lower air passages 40 and 41 respectively. When the tubular capsule 48 is to be used it may be bent as illustrated in Fig. 14b so that the active substance S is collected in the bendable section 49 and is mainly received in the inner corrugation troughs defined therein. When the closure caps 13 have been removed, one end of the capsule 48 may be inserted into the outer end of the air flow passage 30 of the tubular body 29 as illustrated in Fig. 15. Now, the inhaler is ready for use in a manner previously described. When the active substance S from the capsule 48 has been inhaled, the capsule may be discarded and a new capsule is used for the next inhalation.

Fig. 16 shows a tubular inhaler 10 in which the section 12 has a number of annular corrugations. Figs. 16a and 16b illustrate the rectilinear inhaler prior to use and the bent inhaler made ready for use, respectively. As shown in Fig. 16c the corrugations are of a type which is substantially saw tooth shaped in an axial sectional view. Thus, the valleys 50 and peaks 51 of the corrugations are relatively sharp.

In the embodiment shown in Fig. 17 the tubular inhaler 10 is stored in a condition in which the inhaler 10 is bent to a position in which the free ends of the inhaler are positioned closely adjacent, and the inhaler with the active substance therein is maintained in this position by means of a single closure member 52 which is closing both of the free ends of the inhaler. The closure member may, for example, be in the form of a pair of closure caps which are interconnected by a connecting portion which may be formed integrally with the closure caps. This embodiment secures that both of the opposite ends of the tubular inhaler 10 are opened before the inhaler can be used.

It should be understood that various amendments and modifications of the embodiments described above and shown in the drawings could be made without departing from the scope of the present invention. Thus, features described in connection with any of the embodiments shown could also be used in connection with one or more of the other embodiments shown. As an example, any of the embodiments may contain a sieve or grid 38 or other kinds of means for retaining larger particles. Furthermore, any of the tubular bodies shown may comprise separate passages for "false" or enveloping air. Similarly, any of the embodiments of the tubular body may be provided with a compressible bulb 15 as shown in Fig. 1d or any other kind of means for providing compressed air.

## Claims

1. An inhaler, comprising a one piece tubular body (11), which has a mouthpiece section and is intended to be used only once, and which defines an air flow passage therein, the tubular body having substantially rectilinear sections, and a single dose only of an active, inhalable, particulate substance (S) being arranged within the air flow passage, said dose being sealed or closed in relation to the ambient atmosphere by closure means (13) which are to be removed or opened by a user prior to use,
characterized in that the tubular body (11) comprises a curved and/or bendable section (12, 17, 27, 46) intermediate of the rectilinear sections, and in that the cross-sectional area of the flow passage defined in the tubular body (11) does not exceed 75 mm².

2. An inhaler according to claim 1, wherein the curved and/or bendable section (12, 17) of the tubular body (11) comprises peripherally extending corrugations.

3. An inhaler according to claim 2, wherein the tubular body (11) comprises a pair of corrugated bendable sections (12, 46), which are spaced by a non-corrugated rectilinear tubular section (47), so that the tubular body (11) may be bent into a substantial S-shape.

4. An inhaler according to claim 2 or 3, wherein the corrugations of the corrugated bendable section or sections (12, 46) are of the type which in an axial sectional view defines relatively sharp valleys (50) and peaks (51).

5. An inhaler according to claim 4, wherein the corrugations are substantially saw tooth shaped in an axial sectional view.

6. An inhaler according to any of the claims 1-5, wherein the cross-sectional area of the flow passage does not exceed 70 mm² and is preferably less than 50 mm².

7. An inhaler according to claim 6, wherein the cross-sectional area of the flow passage is 7-35 mm², preferably about 20 mm².

8. An inhaler according to any of the claims 1-7, wherein the flow passage has a substantially circular cross-section, the inner diameter of the flow passage being substantially the same along the length of the tubular body.

9. An inhaler according to claim 8, wherein the inhaler is formed similar to a drinking straw.

10. An inhaler according to any of the claims 2-9, wherein the bottoms of the corrugations are provided with codes for assisting in obtaining a bend suitable for the individual user.

11. An inhaler according to any of the claims 1-10, further comprising means (16) for imparting to air flowing through the flow passage a rotational movement about the longitudinal axis of the flow passage.

12. An inhaler according to any of the claims 1-11, wherein the mouthpiece section has a length so as to extend during use from the teeth (24) of the user to a position adjacent to the root of the user's tongue.

13. An inhaler according to any of the claims 1-12, further comprising a bite piece (18) formed on the outer surface of the tubular body (11, 29) for engaging with the upper jaw teeth (24) of the user so as to position the inhaler in the oral cavity (14) of the user.

14. An inhaler according to claim 13, wherein the bite piece (18) is removably mounted on the tubular body (11).

15. An inhaler according to claim 13 or 14, wherein the shape of the bite piece (18) is adapted to the teeth (24) of the individual user.

16. An inhaler according to any of the claims 1-15, wherein the tubular body is moveable from a retracted storage condition to an extended condition of use.

17. An inhaler according to claim 16, wherein the tubular body is provided with peripheral corrugations along a major part of its length so as to allow longitudinal stretching of the tubular body.

18. An inhaler according to any of the claims 1-17, wherein the closure means comprise a pair of cap members (13) removably mounted at opposite ends of the tubular body (11).

19. An inhaler according to any of the claims 1-18, wherein the free ends of the tubular body (11) are positioned closely adjacent, said free ends being closed by a common removable closure member.

20. An inhaler according to claim 18 or 19, wherein at least one of the cap members or closure members (13) is made from a transparent material.

21. An inhaler according to any of the claims 1-20, wherein the tubular body (11) is at least partly made from a transparent material.

22. An inhaler according to any of the claims 1-21, wherein the mouthpiece section of the tubular body is adapted to be inserted into a nostril of a user.

23. An inhaler according to claim 22 comprising a pair of tubular bodies and a connecting part for interconnecting the same, said one end or mouthpiece end of said pair of tubular bodies being arranged in spaced relationship so that said ends may be inserted into the nostrils of a user.

## Patentansprüche

1. Ein Inhalator mit einem einstückigen rohrförmigen Körper (11), der einen Mundstückabschnitt hat und zum Einmalgebrauch gedacht ist und der in sich einen Luftströmungskanal definiert, wobei der rohrförmige Körper im wesentlichen geradlinige Abschnitte hat und sich nur eine einzige Dosis einer aktiven, inhalierbaren Substanz (S) in Pulverform innerhalb des Luftströmungskanals befindet, welche Dosis gegenüber der Umgebungsatmosphäre durch einen Verschluß (13) versiegelt oder verschlossen ist, welcher von einem Benutzer vor der Anwendung zu entfernen oder zu öffnen sind,
dadurch gekennzeichnet, daß der rohrförmige Körper (11) einen gekrümmten und/oder biegbaren Abschnitt (12, 17, 27, 46) zwischen den geradlinigen Abschnitten hat, und daß die Querschnittsfläche des Strömungskanals, die der rohrförmige Körper (11) bestimmt, 75 mm² nicht übersteigt.

2. Ein Inhalator nach Anspruch 1, bei dem der gekrümmte und/oder biegbare Abschnitt (12, 17) des rohrförmigen Körpers (11) eine sich in Umfangsrichtung erstreckende Riffelung aufweist.

3. Ein Inhalator nach Anspruch 2, bei dem der rohrförmige Körper (11) ein Paar von geriffelten biegbaren Abschnitten (12, 46) aufweist, welche durch einen nicht geriffelten geradlinigen rohrförmigen Abschnitt (47) voneinander beabstandet sind, so daß der rohrförmige Körper (11) im wesentlichen in S-Form biegbar ist.

4. Ein Inhalator nach Anspruch 2 oder 3, bei dem die Riffelung des geriffelten biegbaren Abschnittes oder der Abschnitte (12, 46) von einem Typ ist, der in axialer Schnittansicht relativ scharfe Täler (50) und Spitzen (51) aufweist.

5. Ein Inhalator nach Anspruch 4, bei dem die Riffelung in axialer Schnittansicht im wesentlichen sägezahnförmig ist.

6. Ein Inhalator nach einem der Ansprüche 1-5, bei dem die Querschnittsfläche des Strömungskanales 70 mm² nicht übersteigt und bevorzugt kleiner als 50 mm² ist.

7. Ein Inhalator nach Anspruch 6, bei dem die Querschnittsfläche des Strömungskanales 7-35 mm², vorzugsweise ungefähr 20 mm² beträgt.

8. Ein Inhalator nach einem der Ansprüche 1-7, bei dem der Strömungskanal im wesentlichen kreisförmigen Querschnitt hat und der Innendurchmesser des Strömungskanales über die Länge des rohrförmigen Körpers im wesentlichen gleich ist.

9. Ein Inhalator nach Anspruch 8, bei dem der Inhalator ähnlich wie ein Trinkstrohhalm geformt ist.

10. Ein Inhalator nach einem der Ansprüche 2-9, bei dem die Bodenflächen der Riffelung mit einer Codierung versehen sind, um beim Erhalt einer Biegung, welche für den einzelnen Benutzer geeignet ist, zu unterstützen.

11. Ein Inhalator nach einem der Ansprüche 1-10, weiterhin mit Mitteln (16), mit welchen der durch den Strömungskanal strömenden Luft ein Drall um die Längsachse des Strömungskanales aufgeprägt wird.

12. Ein Inhalator nach einem der Ansprüche 1-11, bei dem der Mundstückabschnitt eine solche Länge hat, daß er sich während des Gebrauchs von den Zähnen (24) des Benutzers zu einer Stelle neben der Zungenwurzel des Benutzers erstreckt.

13. Ein Inhalator nach einem der Ansprüche 1-12, weiterhin mit einem Beißstück (18), welches an der äußeren Oberfläche des rohrförmigen Körpers (11, 29) für eine Anlage mit den Oberkieferzähnen (24) des Benutzers ausgebildet ist, um den Inhalator in der Mundhöhle (14) des Benutzers zu positionieren.

14. Ein Inhalator nach Anspruch 13, bei dem das Beißstück (18) entfernbar an dem rohrförmigen Körper (11) angeordnet ist.

15. Ein Inhalator nach Anspruch 13 oder 14, bei dem die Form des Beißstückes (18) an die Zähne (24) des jeweiligen Benutzers angepaßt ist.

16. Ein Inhalator nach einem der Ansprüche 1-15, bei dem der rohrförmige Körper von einem eingezogenen Aufbewahrungszustand in einen ausgestreckten Benutzungszustand beweglich ist.

17. Ein Inhalator nach Anspruch 16, bei dem der rohrförmige Körper mit einer umfangsseitigen Riffelung entlang eines Hauptteiles seiner Länge versehen ist, um eine Strekkung des rohrförmigen Körpers in Längsrichtung zu erlauben.

18. Ein Inhalator nach einem der Ansprüche 1-17, bei dem der Verschluß ein Paar Kappenteile (13) aufweist, welche an gegenüberliegenden Enden des rohrförmigen Körpers (11) entfernbar angeordnet sind.

19. Ein Inhalator nach einem der Ansprüche 1-18, bei dem die freien Enden des rohrförmigen Körpers (11) nahe einander benachbart angeordnet sind und die freien Enden durch ein gemeinsames entfernbares Verschlußteil verschlossen sind.

20. Ein Inhalator nach Anspruch 18 oder 19, bei dem wenigstens eines der Kappenbauteile oder Verschlußbauteile (13) aus einem transparenten Material gefertigt ist.

21. Ein Inhalator nach einem der Ansprüche 1-20, bei dem der rohrförmige Körper (11) zumindest teilweise aus einem transparenten Material gefertigt ist.

22. Ein Inhalator nach einem der Ansprüche 1-21, bei dem der Mundstückabschnitt des rohrförmigen Körpers dafür ausgelegt ist, in eine Nasenöffnung eines Benutzers eingeführt zu werden.

23. Ein Inhalator nach Anspruch 22 mit einem Paar von rohrförmigen Körpern und einem Verbindungsteil zum Verbinden derselben, bei dem das eine Ende oder Mundstückende des Paares von rohrförmigen Körpern in beabstandeter Beziehung so angeordnet ist, daß die Enden in die Nasenöffnungen des Benutzers einführbar sind.

## Revendications

1. Inhalateur, comprenant un corps tubulaire monobloc (11), qui a une partie formant embouchure et qui est prévu pour être utilisé une seule fois, et qui définit un passage intérieur d'écoulement d'air, le corps tubulaire ayant des parties sensiblement rectilignes, et une dose unique seulement d'une substance active inhalable en particules (S) étant placée dans le passage d'écoulement d'air, ladite dose étant isolée ou fermée par rapport à l'atmosphère ambiante par des moyens de fermeture (13) qui doivent être enlevés ou ouverts par un utilisateur avant utilisation,
caractérisé en ce que le corps tubulaire (11) comprend une partie courbe et/ou pliable (12,17,27,46) située entre les parties rectilignes,et en ce que la section transversale du passage d'écoulement défini dans le corps tubulaire (11) ne dépasse pas 75 mm².

2. Inhalateur selon la revendication 1, dans lequel la partie courbe et/ou pliable (12,17) du corps tubulaire (11) comprend des ondulations s'étendant périphériquement.

3. Inhalateur selon la revendication 2, dans lequel le corps tubulaire (11) comprend deux parties pliables ondulées (12,46) qui sont espacées par une partie tubulaire rectiligne non ondulée (47), de sorte qu'on peut plier le corps tubulaire (11) sensiblement en forme de S.

4. Inhalateur selon la revendication 2 ou 3, dans lequel les ondulations de la partie ou des parties pliables ondulées (12,46) sont du type dans lequel une coupe axiale définit des creux (50) et des crêtes (51) à angle relativement vif.

5. Inhalateur selon la revendication 4, dans lequel les ondulations sont sensiblement en dents de scie dans une vue en coupe axiale.

6. Inhalateur selon une quelconque des revendications 1 à 5, dans lequel la section transversale du passage d'écoulement ne dépasse pas 70 mm² et est de préférence inférieure à 50 mm².

7. Inhalateur selon la revendication 6, dans lequel la section transversale du passage d'écoulement est de 7 à 35 mm² et de préférence de 20 mm² environ.

8. Inhalateur selon une quelconque des revendications 1 à 7, dans lequel le passage d'écoulement a une section transversale sensiblement circulaire, le diamètre intérieur du passage d'écoulement étant sensiblement le même sur toute la longueur du corps tubulaire.

9. Inhalateur selon la revendication 8, dans lequel l'inhalateur est de forme similaire à une paille pour boisson.

10. Inhalateur selon une quelconque des revendications 2 à 9, dans lequel les fonds des ondulations portent des codes pour faciliter l'obtention d'une courbure appropriée pour l'utilisateur individuel.

11. Inhalateur selon une quelconque des revendications 1 à 10, comprenant en outre des moyens (16) pour communiquer, à l'air qui circule dans le passage d'écoulement, un mouvement de rotation autour de l'axe longitudinal du passage d'écoulement.

12. Inhalateur selon une quelconque des revendications 1 à 11, dans lequel la partie d'embouchure a une longueur telle qu'elle s'étend, pendant l'utilisation, des dents (24) de l'utilisateur à une position adjacente à la racine de la langue de l'utilisateur.

13. Inhalateur selon une quelconque des revendications 1 à 12, comprenant en outre une pièce à mordre (18) formée sur la surface extérieure du corps tubulaire (11,29) pour engagement avec les dents de la mâchoire supérieure (24) de l'utilisateur afin de positionner l'inhalateur dans la cavité orale (14) de l'utilisateur.

14. Inhalateur selon la revendication 13, dans lequel la pièce à mordre (18) est montée de façon amovible sur le corps tubulaire (11).

15. Inhalateur selon la revendication 13 ou 14, dans lequel la forme de la pièce à mordre (18) est adaptée aux dents (24) de l'utilisateur individuel.

16. Inhalateur selon une quelconque des revendications 1 à 15, dans lequel le corps tubulaire est déplaçable d'un état rétracté de stockage à un état d'utilisation en extension.

17. Inhalateur selon la revendication 16, dans lequel le corps tubulaire comporte des ondulations périphériques le long d'une majeure partie de sa longueur, afin de permettre un étirement longitudinal du corps tubulaire.

18. Inhalateur selon une quelconque des revendications 1 à 17, dans lequel les moyens de fermeture comprennent une paire de capuchons (13) montés de façon amovible aux extrémités opposées du corps tubulaire (11).

19. Inhalateur selon une quelconque des revendications 1 à 18, dans lequel les extrémités libres du corps tubulaire (11) sont placées l'une près de l'autre, lesdites extrémités libres étant fermées par un élément de fermeture amovible commun.

20. Inhalateur selon la revendication 18 ou 19, dans lequel au moins un des capuchons ou éléments de fermeture (13) est fabriqué en une matière transparente.

21. Inhalateur selon une quelconque des revendications 1 à 20, dans lequel le corps tubulaire (11) est au moins en partie fabriqué en une matière transparente.

22. Inhalateur selon une quelconque des revendications 1 à 21, dans lequel la partie d'embouchure du corps tubulaire est prévue pour être insérée dans une narine d'un utilisateur.

23. Inhalateur selon la revendication 22, comprenant deux corps tubulaires et une partie de connexion pour les interconnecter, ladite une extrémité ou extrémité d'embouchure d'un desdits deux corps tubulaires étant espacée de celle de l'autre corps tubulaire d'une manière telle que lesdites extrémités peuvent être insérées dans les narines d'un utilisateur.
